(19) Europäisches Patentamt
European Patent Office
Office européen des brevets

(11) EP 3 883 364 B1

(12) FASCICULE DE BREVET EUROPEEN

(45) Date de publication et mention
de la délivrance du brevet:
05.07.2023 Bulletin 2023/27

(21) Numéro de dépôt: 19804730.0

(22) Date de dépôt: 20.11.2019

(51) Classification Internationale des Brevets (IPC):
*A01H 17/00* (2006.01)    *A01G 18/10* (2018.01)

(52) Classification Coopérative des Brevets (CPC):
A01G 18/10; A01H 17/00

(86) Numéro de dépôt international:
PCT/EP2019/081881

(87) Numéro de publication internationale:
WO 2020/104501 (28.05.2020 Gazette 2020/22)

(54) **PROCÉDÉ DE FABRICATION D'UN INOCULUM DE MYCORHIZE ET INOCULUM OBTENU**

VERFAHREN ZUR HERSTELLUNG VON MYKORRHIZA INOKULUM UND ERHALTENES
INOKULUM

METHOD FOR PRODUCING A MYCORRHIZA INOCULUM AND INOCULUM OBTAINED

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR

(30) Priorité: 20.11.2018  FR 1871637

(43) Date de publication de la demande:
29.09.2021  Bulletin 2021/39

(73) Titulaire: Mycophyto
06250 Mougins (FR)

(72) Inventeur: LIPUMA, Justine
06220 Vallauris (FR)

(74) Mandataire: Decobert, Jean-Pascal
Hautier IP
20, rue de la Liberté
06000 Nice (FR)

(56) Documents cités:
FR-A1- 3 029 737    US-A- 4 294 037

• Marie Chave ET AL: "Multiplier des champignons
mycorhiziens sur son exploitation (suite de la
fiche 1)", , 31 décembre 2017 (2017-12-31),
XP055607336, Extrait de l'Internet:
URL:http://geco.ecophytopic.fr/documents/2
0182/21720/pdf_Multiplier_des_champignons_
mycorhiziens_sur_son_exploitation_1.pdf
[extrait le 2019-07-19]

**Description**

DOMAINE TECHNIQUE

L'invention concerne un procédé de fabrication d'un inoculum de mycorhizes et l'inoculum ainsi obtenu.
Elle trouve son application dans le domaine agronomique dans un but d'améliorer la croissance et la résistance aux stress (biotiques et abiotiques) d'espèces végétales. Cette invention s'intègre dans un contexte global de transition agricole impliquant un changement des pratiques agricoles pour tendre vers des modèles de culture moins dépendants en intrants (eau, engrais, pesticides).

ÉTAT DE LA TECHNIQUE

L'agriculture est une activité particulièrement gourmande en pesticides et en engrais. Les évolutions de l'opinion publique et des réglementations contraignent les agriculteurs à trouver des solutions alternatives, parmi elles on connait l'utilisation du microbiome et en particulier de Champignons Mycorhiziens Arbusculaires (CMA) capables de mettre en place des interactions symbiotiques avec les plantes en formant des mycorhizes.
Cependant, ces champignons sont présents en très faibles quantités dans les sols cultivés.
La fiche technique "Multiplier des champignons mycorhiziens sur son exploitation (suite de la fiche 1)", de l'Institut National de la Recherche Agronomique (INRA), publié sur la page Internet https://geco.ecophytopic.fr/documents/20182/21720/ pdf_Multiplier_des_champignons_mycorhiziens_sur_son_exploitation_1.pdf est une des solutions connues. D'autres solutions connues à ce jour proposent des spores de CMA génériques livrées sous forme de granulés, de poudre ou de solution. Ces CMA ne sont pas adaptés à des conditions de vie réelle, et ne sont pas spécifiques à certains sols ni plantations. En effet, on dénombre environ 200 espèces de CMA et ne sont aujourd'hui commercialisées que 2 à 3 souches pour tous les sols et une gamme très vastes de plantes cibles. Par ailleurs, le temps de cultures pour obtenir un nombre de spores suffisant est actuellement de l'ordre de 3 mois ce qui présente des inconvénients de réactivité et impliquent des couts élevés. Enfin, le niveau de survie des CMA ainsi produits une fois déversés sur les terrains cibles est particulièrement faible. Par ailleurs l'utilisation de souches génériques pose la question d'une possibilité d'invasion de ces souches et de perturbations des communautés biologiques autochtones. Il existe donc le besoin de proposer une solution pour utiliser des CMA de manière efficace dans l'agriculture.

RÉSUMÉ

Pour atteindre cet objectif, la présente invention propose un procédé de fabrication d'un inoculum d'au moins une mycorhize indigène, l'inoculum pouvant comprendre des micro-organismes divers d'intérêt agronomique tels que des champignons mycorhiziens, en particulier du phylum de Glomeromycota et/ou des PGPR (de l'anglais Plant Growth Promoting Rhizobacteria qui sont des bactéries de la rhizosphère bénéfiques à la croissance et à la santé des plantes) et/ou des bactéries fixatrices d'azotes de types Rhizobiacea caractérisé en ce qu'il comprend :

une étape d'obtention d'un substrat amendé comprenant des spores isolées d'un prélèvement de sol d'intérêt qui sont mélangées avec un substrat, le substrat étant, avantageusement adapté pour être compatible avec le prélèvement de sol et présente une texture adaptée à la culture, avantageusement un mélange de sable fin et de perlite, une étape de plantation et de croissance successives d'au moins une première plante piège et d'une deuxième plante piège, distincte de la première plante piège, dans le substrat amendé, une étape d'arrosage de la plantation de la première plante piège et de la deuxième plante piège une fois tous les deux jours avec de l'eau osmosée en alternance avec un milieu nutritif avantageusement pauvre en phosphate, ladite étape de plantation et de croissance comprenant la plantation et la croissance d'une première plante piège, puis la suppression des parties aériennes et un découpage des racines de la première plante piège et réintroduction desdites racines découpées dans le substrat additionné, puis la plantation et la croissance d'une deuxième plante piège, distincte de la première plante piège, dans le substrat amendé comprenant les racines découpées de la première plante piège, puis la suppression des parties aériennes et un découpage des racines de la deuxième plante piège et réintroduction desdites racines découpées dans le substrat additionné comprenant les racines découpées de la première plante piège pour obtenir l'inoculum.

Selon un autre aspect, l'invention concerne un inoculum comprenant un substrat comprenant des parties racinaires mycorhizées d'une première plante piège, des parties racinaires mycorhizées d'une deuxième plante piège, distincte de la première plante piège, et des spores de CMA et présentant une hygrométrie inférieure ou égale à 15%.
Les champignons mycorhizens permettent aux plantes de développer leurs racines et ainsi de multiplier jusqu'à 1000 fois la surface d'échange entre la plante et le sol. Elles peuvent mieux s'enraciner, et chercher les nutriments essentiels à leur croissance beaucoup plus loin. Le demandeur a pu constater de 30 à 60% en plus de poids frais de racines sur des lavandes après inoculation et culture durant 2 mois en comparaison avec des plants témoins. Elles vont ainsi pouvoir puiser plus efficacement des nutriments. Cette activité est la fonction première des mycorhizes, notamment le phosphore, le cuivre, le calcium, le potassium, le magnésium, le zinc, etc. Les champignons mycorhiziens interviennent également dans la décomposition de matières organiques, qu'elles soient végétales ou animales ; et ainsi donnent accès aux plantes à d'autres minéraux, le phosphate en premier. Les mycorhizes s'associent à des bactéries du sol pour dissoudre des minéraux et rendre accessible le phosphore aux plantes. Cette capacité de réguler le flux de phosphore du sol à la plante est la principale fonction de la mycorhize dans les échanges symbiotiques. Dans le cas des légumineuses, la symbiose mycorhizienne est généralement couplée à une autre symbiose, l'association avec les bactéries du genre rhizobium (fixation de l'azote).

L'augmentation de la surface racinaire fait aussi un effet barrière contre des pathogènes, et permet ainsi de protéger la plante. La présence de mycorhize limite également les besoins en engrais de la plante. La difficulté de la production de ces CMA réside dans le fait qu'ils soient des organismes biotrophes obligatoires. En d'autres termes, ces CMA ne sont pas capables de réaliser la totalité de leur cycle de développement sans la mise en place d'une symbiose avec la plante.

Le procédé selon l'invention permet de multiplier rapidement les CMA présents dans un échantillon de sol cible et d'assurer une large biodiversité des CMA en veillant à conserver l'ensemble des souches présentes dans l'échantillon de départ, c'est à dire le prélèvement de sol.

En effet, le procédé permet à partir du prélèvement d'un échantillon de sol cible, c'est-à-dire sur un terrain donné, de débuter le processus de multiplication directement à partir de cet échantillon pour la production d'un inoculum spécifique, adapté au terrain ainsi qu'aux plantes cibles. L'inoculum produit permet l'utilisation de CMA indigènes.

De plus, l'utilisation successive de deux plantes pièges distinctes préserve la biodiversité initiale du prélèvement de sol. Le choix de planter des plantules de plantes pièges permet d'optimiser les contacts entre les CMA issus du prélèvement de sol et la plante au niveau des racines. Enfin, l'arrosage des semis est adapté pour apporter des nutriments suffisants à la plante en croissance sans excès nuisibles au développement de mycorhize. En effet, le choix de l'alimentation des plantes pièges est primordial pour permettre une croissance optimale des mycorhizes et un maintien de l'interaction symbiotique. La plante va rejeter la mycorhization en cas d'ajout d'intrants trop important. La mycorhization est une opération coûteuse en énergie pour la plante, celle-ci la réalisera donc qu'en cas de besoin.

## DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement :
Avantageusement, la première plante piège est une légumineuse, de préférence la luzerne ou le trèfle.

Avantageusement, la deuxième plante piège est une céréalière, de préférence l'orge ou le sorgho.

Avantageusement, le substrat comprend 2/3 de sable et 1/3 de perlite en volume du volume total de sable et de perlite.

Avantageusement, la première plante piège et la deuxième plante piège plantées dans le substrat amendé sont des plantules obtenues par récupération de graines germées.

Avantageusement, les graines germées sont stérilisées préférentiellement à 120°C pendant 20 min et mise en culture des graines germées stérilisées sur un milieu de culture à base d'Agar.

Avantageusement, l'arrosage débute une fois la germination de la plantule débutée, de préférence trois jours après le semis de la plantule dans le substrat amendé.

Avantageusement, le procédé ne comprend pas d'apport de micro-organisme non indigène.

Avantageusement, le procédé ne comprend pas d'apport de mycorhize, ni de spore de CMA non indigène pour obtenir l'inoculum.

Avantageusement, le procédé comprend en outre une étape de séchage de l'inoculum aboutissant à un inoculum présentant une hygrométrie inférieure ou égale à 15%, de préférence, l'étape de séchage est réalisée après la réintroduction dans le substrat des racines découpées de la deuxième plante piège.

Avantageusement, l'étape de séchage comprend un séchage de l'inoculum pendant 24 h à température ambiante.

Avantageusement, le procédé ne comprend pas d'apport de levures ou bactéries non indigènes pour obtenir l'inoculum. Il n'y a pas d'ajout de façon exogène de bactéries, mais le procédé peut permettre de multiplier également des bactéries indigènes du sol. Une des caractéristiques des réseaux mycorhiziens est de permettre le développement de populations bactériennes spécifiques. Par exemple, il peut y avoir un enrichissement de bactéries fixatrices d'azote via l'utilisation de plante piège notamment de type légumineuses. Mais cet apport est indirect et lié au prélèvement de sol initial,

Avantageusement, le procédé ne comprend pas d'étape de multiplication *in vitro* de mycorhizes ou de spores.

Sera définie par mycorhize une association symbiotique d'un champignon avec les racines d'une plante.

On définit par plante 'piège' une plante utilisée pour 'piéger' les CMA présents dans le sol où l'on souhaite cultiver une plante d'intérêt.

Sera définie par spore un organe de stockage et de propagation des CMA. Elle germe et donne naissance à des filaments mycéliens.

Sera définie par symbiose une relation entre deux organismes hétérospécifiques (espèces différentes) qui se traduit par des effets bénéfiques aussi bien pour l'un que pour l'autre.

On entend par indigène que l'élément est présent à l'origine dans le prélèvement de sol effectué. On entend par non-indigène, un élément exogène c'est à dire qui est introduit artificiellement.

L'invention concerne un procédé de fabrication d'un inoculum. Sera défini par inoculum un échantillon qui comprend au moins une souche de champignons arbusculaires mycorhiziens et une mycorhize.

Le procédé comprend deux phases : l'isolement et la production. La première phase d'isolement est destinée à isoler du sol un échantillon comprenant moins un micro-organisme présentant un intérêt agronomique. Préférentiellement, on entend par micro-organisme présentant un intérêt agronomique une souche de champignon arbusculaire mycorhizien indigène et /ou des spores et/ou une mycorhize.

Avantageusement, le procédé débute par un prélèvement de sol d'intérêt. C'est à dire du sol dans lequel l'espèce végétale que l'on souhaite mycorhizer pousse. Selon un mode de réalisation, on prélève une quantité de sol comprise entre 200 et 600 g. À titre d'exemple, sur un terrain de 1Ha, 10 prélèvements sont en moyenne réalisés afin d'obtenir une représentation globale du terrain de culture. Des analyses physico-chimiques sont réalisées pour définir l'hétérogénéité ou l'homogénéité de la culture. Le potentiel mycorhizien du sol initial est mesuré et testé avant production selon la technique du Most Probable Number (MPN) selon Alexander M. (1965) Most probable number method for microbial populations. In Methods of soil analysis. Part 2. Chemical and microbiological properties. Edited by C. A. Black. American Society of Agronomy, Madison, Wisconsin. pp. 1467-1472.Notamment, le taux de mycorhization des systèmes racinaires (coloration de racines, dénombrement des structures d'infections) ainsi que le nombre de spores contenu dans le substrat de production (filtration et dénombrement) sont mesurés. La valeur de ce potentiel va conditionner le temps de production de l'inoculum. L'historique de la parcelle ainsi que la nature des plantes cibles va également influencer sur le choix des plantes pièges dans le procédé de production.

Un des avantages de l'invention est de débuter le procédé à partir d'un échantillon cible comprenant préférentiellement plusieurs souches de CMA dite indigènes, car issues naturellement du milieu à mycorhizer, contrairement à des procédés connus qui utilisent des souches exogènes issues de banques ou de culture en laboratoire. À titre d'exemple non limitatif, la au moins une souche de champignons arbusculaires mycorhiziens présente dans l'échantillon de sol appartient au phylum des *Glomeromycota* tels que. : *Glomus taiwanense, Gigaspora margarita et Funneliformis geosporum* (Liste non exhaustive). Sur un prélèvement de sol, on dénombre classiquement en moyenne entre 5 et 10 souches différentes.

La deuxième phase du procédé concerne la production. Le prélèvement de sol d'intérêt, avantageusement obtenu à l'étape précédente, est mélangé avec un substrat. Un substrat dit substrat additionné est obtenu. Le substrat est configuré pour être à la fois inerte et proche des caractéristiques du sol cible de sorte à assurer une croissance satisfaisante. Avantageusement, le substrat comprend un mélange de sable et de perlite.

Sera défini par sable, préférentiellement un sable fin de type B5 issu de carrières.

Sera définie par perlite des matériaux inertes et imputrescibles, au pH quasiment neutre, très légers, hydrophiles. Préférentiellement, le substrat comprend 2/3 de sable fin et 1/3 perlite. Selon une possibilité, le substrat peut comprendre d'autres constituants secondaires tels que des fibres par exemple. Préférentiellement, les constituants secondaires représentent moins de 1/3 du volume total du substrat. De la fibre de roche ou de coco peut être ajoutée permettant dans certains cas d'apporter une texture plus fibreuse au sol selon le substrat final des plantes cibles ou la nature du prélèvement initial.

Selon une possibilité, le substrat amendé comprend entre 400 et 500g de prélèvement de sol pour 1 à 1,5kg de substrat.

Selon un mode de réalisation, au moins une plante piège est cultivée, préférentiellement pour obtenir une plantule. Préférentiellement, le procédé selon invention comprend au moins deux plantes pièges distinctes qui sont avantageusement utilisées successivement. Une première plante piège est avantageusement dite « plante pionnière », On entend par pionnière qu'elle est la première à coloniser un milieu et permet de mettre en place des conditions plus idéales pour que les autres strates géologiques se fassent. Selon un mode de réalisation, les plantes pièges sont choisies parmi des légumineuses et des oléagineuses céréalières. À titre d'exemple préféré, la première plante piège est une légumineuse, notamment choisie parmi le trèfle ou la luzerne. La première plante piège est choisie avantageusement pour avoir une croissance rapide pour atteindre un stade de plante avec des racines aptes à former rapidement des mycorhizes avec les CMA du substrat amendé. Ces légumineuses sont également intéressantes, car elles sont considérées comme engrais vert grâce à leur apport en azote au sol, d'où leur utilisation en rotation des cultures. À titre d'exemple préféré, la deuxième plante piège est une céréalière, notamment choisie parmi le sorgho ou l'orge. La deuxième plante piège est choisie avantageusement pour assurer une multiplication des mycorhizes optimisée. Préférentiellement, les plantes pièges sont choisies pour ne pas être trop sélectives parmi les CMA avec qui elles forment des mycorhizes. Des essais ont été réalisé permettant de comparer différentes plantes pièges : poireaux, oignons, sorgho, orge, luzerne, trèfle. De ces essais il est noté que l'orge permet la plus importante production de spores, il est donc préféré pour une rotation à la suite d'une culture de luzerne.

Les plantules de plantes pièges sont obtenus après récupération de graines de la au moins une plante piège et stérilisation desdites graines. Avantageusement, les graines sont scarifiées par exemple par $HgSO_4$. La scarification permet d'accélérer la germination de la graine de façon chimique ou mécanique. La stérilisation permet de s'assurer de la non contamination des graines par des bactéries ou champignons néfastes. La stérilisation se fait soit par exposition thermique, par exemple en plaçant les graines dans une étuve à 120°C pendant 20 min soit par javellisation de 3 min à 4 min selon le type de graines. Enfin, ces graines sont mises dans l'eau durant 3 à 4 heures.

Les graines stérilisées sont mises en culture préférentiellement sur un milieu de culture à base d'Agar. Ce milieu aqueux favorise la germination et permet de visualiser les plants pour ne replanter que les plants germés

Les graines sont mises à germer sur un milieu Agar par exemple 0,4% à l'obscurité pendant 2 jours. La plantule obtenue de la plante piège est plantée dans un contenant comprenant du substrat amendé. Une vingtaine de plantules sont additionnées à un volume de 5L de substrat.

L'avantage d'utiliser des plantules de plantes pièges et de s'assurer un contact rapide entre les racines de la plantule et les souches CMA contenues dans le substrat amendé.

Selon un mode de réalisation préféré, une première plante piège est plantée dans un contenant comprenant le substrat amendé pendant une période de croissance comprise entre 10 et 20 jours préférentiellement 15 jours. La rotation s'effectue après le développement végétatif de la plante et avant la période de floraison. Ensuite, les parties aériennes de la première plante piège sont coupées et les racines sont coupées par exemple à l'aide d'un sécateur au préalable stérilisé en morceaux de 1 à 2cm et réintroduites dans le substrat amendé.

Une plantule de la deuxième plante piège obtenue comme décrit précédemment est ensuite plantée dans le contenant comprenant le substrat amendé et les parties racinaires découpées de la première plante piège.

La deuxième plante piège est mise en croissance pendant une période de croissance comprise entre 10 et 20 jours préférentiellement 15 jours. Les parties aériennes sont retirées ensuite, les racines sont coupées en petits morceaux sur le même modèle que la première plante piège et replacées dans le substrat amendé pour former l'inoculum destiné à être utilisé.

Au cours des périodes de croissance de la première et deuxième plante piège, un arrosage est réalisé. L'arrosage est défini de sorte qu'il n'y est pas de stagnation d'eau dans le contenant.

Selon un mode de réalisation préféré, l'arrosage débute 3 jours après la plantation des plantules. Préférentiellement, l'arrosage débute lorsque la germination est finie et que la plantule est devenue une plante. On entend par plantule une plante embryonnaire quand les cotylédons sont encore fonctionnels.

Selon un mode de réalisation, l'arrosage consiste en un apport d'eau préférentiellement osmosée. Préférentiellement, l'arrosage consiste également en un apport de milieu nutritif complet, préférentiellement un milieu de Culture d'Organe Racinaire ou ROC (pour Root Organ Cultures en anglais) de Bécard et Fortin de 1988 adapté au contexte ici de production d'inoculum avec utilisation de plantes pièges. Avantageusement, l'apport d'eau et de milieu nutritif se fait de manière alternée. C'est-à-dire un jour, l'arrosage se fait avec de l'eau osmosée et le jour suivant l'arrosage se fait avec un milieu nutritif. Préférentiellement, le milieu nutritif est choisi pour être pauvre en phosphate et azote de sorte que les CMA et la plante piège soient poussés à former une mycorhize. Ce mode de réalisation se fait pour chaque plante piège qui est plantée dans le substrat amendé.

Selon une possibilité, l'arrosage débute par 0,5 à 2% d'eau en volume par rapport au volume du contenant, préférentiellement 1% d'eau et avantageusement pendant 1 à 3 jours préférentiellement 2 jours. L'arrosage se poursuit par 3% à 7% d'eau en volume par rapport au volume du contenant, préférentiellement 5% d'eau et avantageusement pendant 1 à 3 jours préférentiellement 2 jours.

Avantageusement, la croissance des plantes pièges se fait en serre de sorte à contrôler les conditions humidité, d'ensoleillement et de température. Les conditions sont sélectionnées pour être à la fois optimales pour les plantes pièges ainsi que pour la mycorhize. Les conditions sont choisies dans un intervalle de valeurs pour satisfaire aux deux types de plantes différentes : Légumineuse/Céréale.

À titre d'exemple, les conditions climatiques sont:

- Aération 24 - 25°C,

- Écran d'ombrage présentant déploiement de 500 W/m2, et reploiement de 400 W/m2

Avantageusement, le contenant recevant la plante piège et le substrat amendé, présente un volume initial de 5l pour 20 plantules. Préférentiellement, le volume du contenant est augmenté au cours de la période de croissance des plantes pièges.

L'inoculum selon l'invention comprend des spores, des parties racinaires mycorhizées d'une première plante piège et des parties racinaires mycorhizées d'une deuxième plante piège.

Cet inoculum sera ensuite par différents procédés inoculés à des plantes cibles ou directement intégré dans un substrat de culture. Les quantités de spores ainsi que les concentrations seront définies dans les protocoles des procédés d'inoculation au cas par cas.

L'inoculum ainsi préparé peut être conservé 2 à 3 mois à 4°C après avoir mis l'ensemble à sécher. Avantageusement, l'inoculum est mis à sécher pendant 20h à 30h, préférentiellement environ 24h à température ambiante, c'est à dire entre 20 et 25°C. Selon l'invention, l'hygrométrie de l'inoculum à l'issue de l'étape de séchage est inférieure ou égale à 15%.

Exemples

Exemple 1

Un prélèvement de 300 grammes de sol est mélangé avec un substrat comprenant 2/3 de sable et 1/3 de perlite pour obtenir 2000 grammes de substrat amendé. Une mise en culture dans le substrat amendé de la première plante piège de type luzerne est réalisée. Après 4 semaines de culture, les parties aériennes de la première plante piège sont supprimées et les racines découpées en petit morceau et réintroduites dans le substrat amendé. Une mise en culture dans le substrat amendé, contenant les racines de la première plante piège découpées en petit morceau, de la deuxième plante piège de type orge est réalisée. Après 4 semaines de culture, les parties aériennes de la deuxième plante piège sont supprimées et les racines découpées en petit morceau et réintroduites dans le substrat amendé pour former l'inoculum.

Exemple 2 : mesure de nombres de spores dans un échantillon de sol.

Un échantillon de sol d'environ 50g est prélevé. L'échantillon ainsi que le pot où il est placé sont pesés. Les spores sont récupérées selon la méthode de tamisage humide : Gerdemann et Nicolson (1963). La terre est reprise dans 50mL d'eau courante. Après 10 à 30s de décantation, l'échantillon est passé à travers une série de 3 tamis (775 ;300 ; 30$\mu$m) submergé par l'eau courante.

Les refus de tamis entre 300 et 30$\mu$m sont récupérés dans 50mL d'eau non stérile et répartis dans deux tubes flacons 50mL. Une solution de saccharose à 2M est ajoutée qsp 40mL. Les tubes sont centrifugés 5min -3000rpm- RT et le surnageant est ensuite filtré par filtration Buchner. Les spores sont alors dénombrées sous la loupe binoculaire.

Selon cette méthode de détermination du nombre de spores, le nombre de spores actives/g est déterminé à différents temps T 1,2,3 et 4 semaines sur des échantillons de sols de l'exemple 1.

Les résultats sont donnés ci-après

T1 semaine : 0,29 spores/g ;

T2 semaines : 0,718 spores/g ;

T3 semaines : 0,8 spores/g ;

T4 semaines : 0,9 spores/g.

Soit une augmentation de 70% de la quantité de spores entre la première semaine et la dernière semaine de mise en culture.

Exemple 3 : Suivi de la production de spore d'un mix de souche indigène avec deux plantes pièges différentes en fonction du temps

Le nombre de spores d'un échantillon de sol est mesuré selon l'exemple 2. Le substrat amendé selon l'invention reçoit une plante piège soit une légumineuse telle que de la luzerne soit une céréalière telle que du sorgho. Au bout de trois semaines et de cinq semaines pour chacune des plantes pièges, le taux de mycorhization des systèmes racinaires (coloration de racines, dénombrement des structures d'infections) ainsi que le nombre de spores contenu dans le substrat de production (filtration et dénombrement) sont mesurés, notamment par la méthode du Most Probable Number (MPN) selon Alexander M. (1965). Les résultats sont donnés dans le tableau ci-dessous.

**Suivi de la production de spore d'un mix de souche indigène dans deux plantes pièges différentes en fonction du temps**

| Nombre de spores - plantes pièges | spores /mg |
|---|---|
| Initiales | 1100 |
| 3 semaines Céréalière | 1150 |
| 3 semaines Légumineuse | 1050 |
| 5 semaines Céréalière | 1550 |
| 5 semaines Légumineuse | 1350 |

On constate que la céréalière permet une multiplication plus importante des spores par rapport à la légumineuse. Ainsi, il est préféré d'utiliser la céréalière après la légumineuse.

Le temps de réalisation d'un cycle de reproduction de spores par des Eumycètes endomycorhiziens (type de CMA) selon les différentes espèces et le type de plante piège est déterminé. La vitesse spécifique de croissance ($\mu$) est calculée afin de déterminer le temps de génération (G) grâce aux formules suivantes :

$$\mu = \frac{\ln(Biomasse/g\ t2) - \ln(Biomasse\ spores/g\ t1)}{t2 - t1}$$

et

$$G = \frac{\ln(2)}{\mu}$$

On constate que des racines de chicorée mises en culture in vitro présente un temps de génération d'une souche CMA de Rhizophagus irregularis de 41 jours ( 6 semaines) alors que l'orge mise en culture selon le procédé décrit à l'exemple 1 après la luzerne comme première plante piège présente un temps de génération d'une souche CMA de Rhizophagus irregularis de 24 jours (3,5 semaines), soit un temps de génération nettement réduit.

Exemple 4

Composition d'un milieu Long-Ashton revu pour production sous serre de plante piège de mycorhizes

| Macronutriments | Concentration mM | Solution mère | ml de solution mère/L de solution finale |
|---|---|---|---|
| MgSO$_4$ *7 H$_2$O | 0,75 | 18% | 1 |
| NaNO$_3$ | 1 | 4,25% | 2 |
| K$_2$SO$_4$ | 1 | 8,7% | 2 |
| CaCl$_2$ *5 H$_2$0 | 2 | 14,7% | 2 |
| Na$_2$HPO$_4$ *12 H$_2$O | 0,0032 | 0,06% | 2 |
| **Microéléments** | | | |
| FeNa-EDTA | 0,025 | 0,46% | 2 |
| MnSO$_4$ *1H$_2$O® | 0,005 | 0,84% | 0,1 |
| CuSO$_4$ *5 H$_2$O | 0,00025 | 0,04% | 0,1 |
| Zn SO$_4$ *7 H$_2$O | 0,0005 | 0,145% | 0,1 |
| H$_3$BO$_3$ | 0,025 | 1,55% | 0,1 |

(suite)

| Microéléments | | | |
|---|---|---|---|
| $Na_2MoO_4$ *$2H_2O$ | 0,0001 | 0,0242% | 0,1 |

## Revendications

1. Procédé de fabrication d'un inoculum d'au moins une mycorhize **caractérisé en ce qu'**il comprend :

   ▪ une étape d'obtention d'un substrat amendé comprenant des spores isolées d'un prélèvement de sol d'intérêt mélangé avec un substrat, le substrat étant un mélange de sable fin et de perlite,
   ▪ une étape de plantation et de croissance successives d'au moins une première plante piège et d'une deuxième plante piège, distincte de la première plante piège, dans le substrat amendé,
   ▪ une étape d'arrosage de la plantation de la première plante piège et de la deuxième plante piège une fois tous les deux jours avec de l'eau osmosée en alternance avec un milieu nutritif pauvre en phosphate,
   ▪ ladite étape de plantation et de croissance comprenant la plantation et la croissance d'une première plante piège, puis la suppression des parties aériennes et un découpage des racines de la première plante piège et réintroduction desdites racines découpées dans le substrat additionné, puis la plantation et la croissance d'une deuxième plante piège, distincte de la première plante piège, dans le substrat amendé comprenant les racines découpées de la première plante piège, puis la suppression des parties aériennes et un découpage des racines de la deuxième plante piège et réintroduction desdites racines découpées dans le substrat additionné comprenant les racines découpées de la première plante piège pour obtenir l'inoculum.

2. Procédé selon la revendication précédente dans lequel la première plante piège est une légumineuse.

3. Procédé selon la revendication précédente dans lequel la légumineuse est de la luzerne ou du trèfle.

4. Procédé selon l'une quelconque des revendications précédentes dans lequel la deuxième plante piège est une céréalière.

5. Procédé selon la revendication précédente dans lequel la céréalière est du sorgho ou de l'orge.

6. Procédé selon l'une quelconque des revendications précédentes dans lequel le substrat comprend 2/3 de sable et 1/3 de perlite en volume du volume total de sable et de perlite.

7. Procédé selon l'une quelconque des revendications précédentes dans lequel la première plante piège et la deuxième plante piège plantées dans le substrat amendé sont des plantules obtenues par récupération de graines germées.

8. Procédé selon la revendication précédente dans lequel l'arrosage débute une fois la germination de la plantule débutée.

9. Procédé selon l'une quelconque des revendications précédentes comprenant en outre une étape de séchage de l'inoculum aboutissant à un inoculum présentant une hygrométrie inférieure ou égale à 15%.

10. Procédé selon la revendication précédente dans lequel l'étape de séchage comprend un séchage de l'inoculum pendant 24 h à température ambiante.

11. Procédé selon l'une quelconque des revendications précédentes ne comprenant pas d'apport de micro-organisme non indigène.

12. Inoculum obtenu par le procédé selon la revendication 9, comprenant un substrat comprenant des parties racinaires mycorhizées d'une première plante piège, des parties racinaires mycorhizées d'une deuxième plante piège, distincte de la première plante piège, et des spores de champignon mycorhizien arbusculaire (CMA) et présentant une hygrométrie inférieure ou égale à 15%.

# EP 3 883 364 B1

**Patentansprüche**

1. Verfahren zur Herstellung eines Inokulums aus mindestens einer Mykorrhiza, **dadurch gekennzeichnet, dass** es Folgendes umfasst:

   • einen Schritt zum Erhalten eines verbesserten Substrats, das Sporen umfasst, die aus einer Bodenprobe von Interesse isoliert wurden, die mit einem Substrat vermischt wurde, wobei es sich bei dem Substrat um eine Mischung aus feinem Sand und Perlit handelt,
   • einen Schritt des aufeinanderfolgenden Pflanzens und Wachstums von mindestens einer ersten Fangpflanze und einer zweiten Fangpflanze, die sich von der ersten Fangpflanze unterscheidet, in dem verbesserten Substrat,
   • einen Schritt des Gießens der Pflanzung der ersten Fangpflanze und der zweiten Fangpflanze einmal alle zwei Tage mit Osmosewasser im Wechsel mit einem phosphatarmen Nährmedium,
   • wobei der Pflanz- und Wachstumsschritt das Pflanzen und Wachstum einer ersten Fangpflanze, dann das Entfernen der oberirdischen Teile und ein Abschneiden der Wurzeln der ersten Fangpflanze und das Wiedereinführen der abgeschnittenen Wurzeln in das zugegebene Substrat und dann das Pflanzen und Wachstum einer zweiten Fangpflanze umfasst, die sich von der ersten Fangpflanze unterscheidet, in das zugegebene Substrat, das die abgeschnittenen Wurzeln der ersten Fangpflanze umfasst, dann das Entfernen der oberirdischen Teile und ein Abschneiden der Wurzeln der zweiten Fangpflanze und Wiedereinführen der abgeschnittenen Wurzeln in das zugegebene Substrat, das die abgeschnittenen Wurzeln der ersten Fangpflanze umfasst, um das Inokulum zu erhalten.

2. Verfahren nach dem vorhergehenden Anspruch, wobei die erste Fangpflanze ein Hülsenfrüchtler ist.

3. Verfahren nach dem vorhergehenden Anspruch, wobei es sich bei dem Hülsenfrüchtler um Luzerne oder Klee handelt.

4. Verfahren nach einem der vorhergehenden Ansprüche, wobei die zweite Fangpflanze eine Getreidepflanze ist.

5. Verfahren nach dem vorhergehenden Anspruch, wobei es sich bei dem Getreide um Sorghum oder Gerste handelt.

6. Verfahren nach einem der vorhergehenden Ansprüche, wobei das Substrat 2/3 Sand und 1/3 Perlit als Volumen des Gesamtvolumens von Sand und Perlit umfasst.

7. Verfahren nach einem der vorhergehenden Ansprüche, wobei die erste Fangpflanze und die zweite Fangpflanze, die in das geänderte Substrat gepflanzt werden, Sämlinge sind, die durch Gewinnung von gekeimten Samen erhalten wurden.

8. Verfahren nach dem vorhergehenden Anspruch, wobei das Gießen beginnt, sobald die Keimung des Sämlings begonnen hat.

9. Verfahren nach einem der vorhergehenden Ansprüche, das außerdem einen Schritt zum Trocknen des Inokulums umfasst, der zu einem Inokulum führt, das eine Luftfeuchtigkeit geringer als oder gleich 15 % aufweist.

10. Verfahren nach dem vorhergehenden Anspruch, wobei der Trocknungsschritt das Trocknen des Inokulums für 24 Stunden bei Raumtemperatur umfasst.

11. Verfahren nach einem der vorhergehenden Ansprüche, das keine Zufuhr von nicht-einheimischen Mikroorganismen umfasst.

12. Inokulum, das durch das Verfahren nach Anspruch 9 erhalten wird, umfassend ein Substrat, das mykorrhizierte Wurzelteile einer ersten Fangpflanze, mykorrhizierte Wurzelteile einer zweiten Fangpflanze, die sich von der ersten Fangpflanze unterscheidet, und Sporen eines arbuskulären Mykorrhizapilzes (CMA) enthält und eine Luftfeuchtigkeit geringer als oder gleich 15 % aufweist.

**Claims**

1. Method for manufacturing an inoculum of at least one mycorrhiza, **characterised in that** it comprises:

• a step of obtaining an amended substrate comprising spores isolated from a sample of soil of interest mixed with a substrate, the substrate being a mixture of fine sand and perlite,

• a step of successive plantation and growth of at least one first trap plant and a second trap plant, distinct from the first trap plant, in the amended substrate,

• a step of watering the plantation of the first trap plant and of the second trap plant once every two days with osmosed water in alternation with a phosphate-depleted nutritive medium,

• said plantation and growth step comprising the plantation and the growth of a first trap plant, then the suppression of the above-ground parts and a cutting of the roots of the first trap plant and reintroduction of said cut roots into the supplemented substrate, and then the plantation and growth of a second trap plant, distinct from the first trap plant, in the amended substrate comprising the cut roots of the first trap plant, and then the suppression of the above-ground parts and cutting of the roots of the second trap plant and reintroduction of said cut roots into the supplemented substrate comprising the cut roots of the first trap plant to obtain the inoculum.

2. Method according to the preceding claim, wherein the first trap plant is a legume.

3. Method according to the preceding claim, wherein the legume is alfalfa or clover.

4. Method according to any one of the preceding claims, wherein the second trap plant is a cereal.

5. Method according to the preceding claim, wherein the cereal is sorghum or barley.

6. Method according to any one of the preceding claims, wherein the substrate comprises 2/3 sand and 1/3 perlite by volume of the total volume of sand and perlite.

7. Method according to any one of the preceding claims, wherein the first trap plant and the second trap plant planted in the amended substrate are plant germs obtained by recovering germinated seeds.

8. Method according to the preceding claim, wherein the watering begins once the germination of the plant germ has begun.

9. Method according to any one of the preceding claims, furthermore comprising a step of drying the inoculum resulting in an inoculum having a moisture content of less than or equal to 15%.

10. Method according to the preceding claim, wherein the drying step comprises a drying of the inoculum for 24 hours at ambient temperature.

11. Method according to any one of the preceding claims, not comprising the addition of any non-indigenous microorganism.

12. Inoculum obtained by the method according to claim 9, comprising a substrate comprising mycorrhyzal root parts of a first trap plant, mycorrhyzal root parts of a second trap plant, distinct from the first trap plant, and arbuscular mycorrhizal fungus (AMF) spores having a moisture content of less than or equal to 15%.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Most probable number method for microbial populations. In Methods of soil analysis. **ALEXANDER M.** Chemical and microbiological properties. American Society of Agronomy, 1965, 1467-1472